(19) Europäisches Patentamt
European Patent Office
Office européen des brevets

(11) Veröffentlichungsnummer: **0 176 798**
**B1**

(12) **EUROPÄISCHE PATENTSCHRIFT**

(45) Veröffentlichungstag der Patentschrift: **18.01.89**

(51) Int. Cl.⁴: **C 07 C 87/60**, A 61 K 7/13

(21) Anmeldenummer: **85111228.4**

(22) Anmeldetag: **05.09.85**

(54) **Aminodiphenylamine und diese enthaltende Haarfärbemittel.**

(30) Priorität: **13.09.84 DE 3433594**

(43) Veröffentlichungstag der Anmeldung: **09.04.86 Patentblatt 86/15**

(45) Bekanntmachung des Hinweises auf die Patenterteilung: **18.01.89 Patentblatt 89/3**

(84) Benannte Vertragsstaaten: **DE**

(56) Entgegenhaltungen:
**DE-A- 2 507 567**
**GB-A- 2 016 013**
**US-A- 4 257 805**

**CHEMICAL ABSTRACTS, Band 101, Nr. 13, 24. September 1984, Seite 621, Nr. 110569d, Columbus, Ohio, US; & JP - A - 59 27 864 (KUMIAI CHEMICAL INDUSTRY CO., LTD.) 14-02-1984**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Rose, David, Dr., Holbeinweg 7, D-4010 Hilden (DE)**
Erfinder: **Lieske, Edgar, Hunsrückenstrasse 40, D-4000 Düsseldorf (DE)**
Erfinder: **Maak, Norbert, Dr., Liebigstrasse 18, D-4040 Neuss (DE)**

**EP 0 176 798 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue, trifluormethylsubstituierte Aminodiphenylamine und deren Verwendung als Oxidationsfarbstoffvorprodukte in Haarfärbemitteln.

Für das Färben von Haaren spielen Oxidationshaarfärbemittel wegen ihrer intensiven Farben und guten Echtheitseigenschaften eine besondere Rolle. Solche Haarfärbemittel enthalten Oxidationsfarbstoffvorprodukte in einem kosmetischen Träger. Als Oxidationsfarbstoffvorprodukte werden Verbindungen eingesetzt, die unter dem Einfluss von Oxidationsmitteln oder von Luftsauerstoff Farbstoffe ausbilden. Man unterscheidet dabei sogenannte Entwicklersubstanzen, die durch oxidative Kupplung untereinander oder mit einer oder mehreren Kupplerkomponenten Farbstoffe bilden, und Kupplersubstanzen, die selbst keine brauchbaren Farbstoffe ausbilden, die aber zusammen mit Entwicklersubstanzen deren Farbton modifizieren und die Farbintensität erhöhen. Als kosmetische Träger für die Oxidationsfarbstoffvorprodukte dienen Cremes, Emulsionen, Gele, Shampoos, Schaumaerosole und andere Zubereitungen, die für die Anwendung auf dem Haar geeignet sind.

Als Entwicklersubstanzen werden üblicherweise primäre, aromatische Amine mit einer weiteren in Para oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als Kupplersubstanzen werden m-Phenylen-diaminderivate, m-Aminophenolderivate, Naphthole, Resorcin und Pyrazolone verwendet.

Gute Oxidationshaarfarbstoffvorprodukte müssen in erster Linie folgende Voraussetzungen erfüllen: Sie sollen bei der oxidativen Kupplung die gewünschten Farbnuancen in ausreichender Intensität ausbilden, sie sollen ein gutes Aufziehvermögen auf menschliches Haar besitzen ohne die Kopfhaut zu stark anzufärben und sie sollen in toxikologischer und dermatologischer Hinsicht unbedenklich sein. Die Haarfärbungen sollen eine hohe Echtheit gegen Licht, Wärme, Schweiss, Reibung und Dauerwellbehandlungen aufweisen.

Aminodiphenylamine sind als Oxidationshaarfarbstoffvorprodukte z.B. aus DE-PS 294 184 und aus DE-OS 2 507 567 bekannt. Die mit diesen Oxidationsfarbstoffvorprodukten herstellbaren Haarfärbemittel befriedigen jedoch nicht in bezug auf die Echtheitseigenschaften der damit erzielbaren Haarfärbungen. Unter dem Einfluss der Wärme kommt es zu unerwünschten Farbänderungen. Viele der bekannten Aminodiphenylamine, z.B. das 2,4-Diamino-4'-aminodiphenylamin, sind mit anderen Kupplern nicht kompatibel, so dass eine gezielte Modifikation der damit erhaltenen, meist blauen Nuancen durch bekannte Gelb- oder Rotkuppler nicht möglich ist.

Gegenstand der Erfindung sind trifluormethylsubstituierte Aminodiphenylamine der allgemeinen Formel

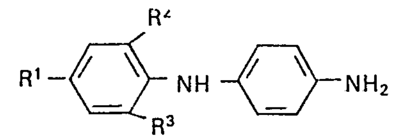

in der $R^1$, $R^2$ und $R^3$ Wasserstoff, eine Alkylgruppe mit 1–4 C-Atomen, eine $NH_2$-Gruppe oder $CF_3$-Gruppe sind, wobei eine dieser Gruppen die $CF_3$-Gruppe und wenigstens eine die $NH_2$-Gruppe darstellt.

Es wurde gefunden, dass diese neuen, trifluormethylsubstituierten Aminodiphenylamine sich als Oxidationsfarbstoffvorprodukte für Haarfärbemittel in besonderer Weise eignen. Bei ihrer Verwendung als Kupplersubstanzen ermöglichen sie mit einer Vielzahl der bekannten Entwicklersubstanzen die Ausbildung intensiver, brillanter Braun- und Rot-Nuancen von hoher Wärmestabilität und Lichtechtheit. Mit bekannten Kupplersubstanzen besteht eine gute Verträglichkeit, so dass auch gezielte Modifikationen der Nuancen bekannter Entwickler-Kuppler-Systeme möglich sind. Die neuen Aminodiphenylamine stellen daher eine wertvolle Bereicherung der Palette der Oxidationshaarfarbstoffvorprodukte dar. Besonders günstige anwendungstechnische Eigenschaften weisen die Verbindungen der allgemeinen Formel I auf, wenn $R^1$ die Trifluormethylgruppe darstellt. Bevorzugt ist das 2,6-Diamino-4-trifluormethyl-4'-aminodiphenylamin. Die mit diesem Aminodiphenylaminen und bekannten Entwicklersubstanzen erzielbaren Haaranfärbungen zeichnen sich durch hohe Echtheitseigenschaften aus.

Die Herstellung der erfindungsgemässen Aminodiphenylamine der allgemeinen Formel I kann z.B. in der Weise erfolgen, dass man eine Verbindung der allgemeinen Formel II

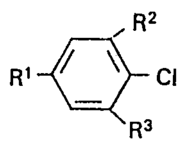

II

in welcher $R^1$, $R^2$ und $R^3$ Wasserstoff, eine Alkylgruppe mit 1–4 C-Atomen, eine $NO_2$-Gruppe oder eine $CF_3$-Gruppe sind, wobei eine dieser Gruppen die $CF_3$-Gruppe und wenigstens eine die $NO_2$-Gruppe darstellt, mit N-Acetyl-p-phenylendiamin in Gegenwart von Natriumhydrogencarbonat und Kupfer (I)-chlorid in einem polaren, inerten Lösungsmittel umsetzt, von dem entstehenden N-Acetyl-Nitrodiphenylamin die Acetalgruppe abspaltet und das Nitrodiphenylamin katalytisch zum Aminodiphenylamin hydriert.

Die Verwendung der trifluormethylsubstituierten Diphenylamine als Oxidationsfarbstoffvorprodukte in Haarfärbemitteln kann in freier Form oder in Form der Salze mit anorganischen oder organischen Säuren, z.B. in Form der Hydrochloride, Sulfate, Phosphate, Acetate, Propionate, Lactate oder Citrate erfolgen. Die Verwendung der Verbindungen erfolgt bevorzugt als Kuppler gemeinsam mit bekannten Entwicklerkomponenten.

Ein weiterer Gegenstand der Erfindung sind daher Haarfärbemittel, enthaltend Oxidationshaarfarbstoffvorprodukte in einem kosmetischen Träger, die als Oxidationsfarbstoffvorprodukte Aminodiphenylamine der allgemeinen Formel I oder deren Salze und die in Oxidationsfärbemitteln üblichen Entwicklersubstanzen enthalten. Zur Modifikation der Nuancen können die erfindungsgemässen trifluormethylsubstituierten Aminodiphenylamine mit bekannten Kuppler gemeinsam verwendet werden. Weiterhin können in solchen erfindungsgemässen Haarfärbemitteln auch direktziehende Haarfarbstoffe, z.B. solche vom Typ der Nitrophenylendiaminderivate, Anthrachinonfarbstoffe oder Indophenole zur Erzielung natürlicher Haarfarbnuancen mit den Oxidationshaarfarbstoffvorprodukten kombiniert eingesetzt werden.

Als Beispiel für in den erfindungsgemässen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diethyl-2-methyl- p-phenylendiamin, N-Ethyl-N-hydroxyethyl- p-phenylendiamin, Chlor-p-phenylendiamin, N,N-Bis-hydroxyethylamino-p-phenylendiamin, Methoxy-p-phenylendiamin, 2,6-Dichlor- p-phenylendiamin, 2-Chlor-6-brom- p-phenylendiamin, 2-Chlor-6-methyl- p-phenylendiamin, 6-Methoxy- 3-methyl- p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1–4 Kohlenstoffatomen darstellt, ferner Diaminopyridinderivate, heterocyclische Hydrazonderivate wie 1-Methylpyrrolidon- (2)-hydrazon, 4-Aminopyrazolonderivate wie 4-Amino-1-phenyl- 3-carbamoylpyrazolon-5, N-Butyl-N-sulfobutyl- p-phenylendiamin, Tetraaminopyrimidine wie 2,4,5,6-Tetraaminopyrimidin, 4,5-Diamino- 2,6-bismethylaminopyrimidin, 2,5-Diamino-4-diethylamino-6-methyl-aminopyrimidin, 2,4,5-Triamino-6-dimethylamino-pyrimidin, 2,4,5-Triamino-6-piperidino-pyrimidin, 2,4,5-Triamino- 6-anilinopyrimidin, 2,4,5-Triamino- 6-morpholino-pyrimidin,2,4,5-Triamino- 6-β-hydroxy-ethylamino-pyrimidin anzuführen.

In den erfindungsgemässen Haarfärbemitteln werden die Entwicklerverbindungen und die Kupplerverbindungen im allgemeinen in etwa äquimolekularen Mengen eingesetzt. Wenn sich auch der äquimolekulare Einsatz als zweckmässig erwiesen hat, so ist es doch nicht nachteilig, wenn einzelne Oxidationsfarbstoffvorprodukte im Überschuss zum Einsatz gelangen. Es ist auch nicht erforderlich, dass die erfindungsgemässen Aminodiphenylamine oder die sonst enthaltenen Oxidationsfarbstoffvorprodukte einheitliche Verbindungen sind. Vielmehr können auch Gemische dieser Verbindungen zum Einsatz gelangen.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemässen Haarfärbemittel werden die Oxidationsfarbstoffvorprodukte in einen geeigneten kosmetischen Träger eingearbeitet. Solche Träger sind zum Beispiel Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind z.B. Netz- und Emulgiermittel wie anionische, nichtionische oder ampholytische Tenside, z.B. Fettalkoholsulfate, Alkansulfonate, α-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z.B. Methyl- oder Hydroxymethylcellulose, Stärke, Fettalkohole, Paraffinöle, Fettsäuren, ferner Parfümöle und haarpflegende Zusätze, wie z.B. wasserlösliche kationische Polymere, Proteinderivate, Pantothensäure und Cholesterin. Die Bestandteile der kosmetischen Träger werden zur Herstellung der erfindungsgemässen Haarfärbemittel in für diese Zwecke üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt. Die Oxidationsfarbstoffvorprodukte werden in Mengen von 0,2 bis 5 Gew.-%, vorzugsweise 1 bis 3 Gew.-% des gesamten Färbemittels in den Träger eingemischt. Die trifluormethylsubstituierten Aminodiphenylamine der allgemeinen Formel I werden in Mengen von 0,05–10 Millimol pro 100 g in die erfindungsgemässen Haarfärbemittel eingesetzt.

Die Anwendung der erfindungsgemässen Haarfärbemittel kann, unabhängig von der Art der kosmetischen Zubereitung, z.B. als Creme, Gel oder Shampoo, im schwach sauren, neutralen oder alkalischen Milieu erfolgen. Bevorzugt ist die Anwendung der Haarfärbemittel in einem pH-Bereich von 8–10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern, ohne ihn jedoch hierauf zu beschränken.

Herstellungsbeispiele

2-Amino-4-trifluormethyl- 4'-aminodiphenylamin- dihydrochlorid (KI)

Stufe 1

Eine Mischung aus 10 g (0,04 Mol) 4-Chlor-3-nitro-benzotrifluorid 6,7 g (0,04 Mol) N-Acetyl-p-phenylendiamin, 4 g (0,05 Mol) Natriumhydrogencarbonat und 0,5 g Kupfer(I)-chlorid in 100 ml Nitrobenzol wurde 8 Stunden lang auf 170 °C erhitzt. Nach Entfernung des Nitrobenzols durch Wasserdampfdestillation wurde das Reaktionsgemisch filtriert und der Niederschlag aus Ethanol umkristallisiert. Es wurden 10,4 g 2-Nitro- 4-trifluormethyl- 4'-acetylamino-diphenylamin mit einem Schmelzpunkt von 177–178 °C erhalten.

Stufe 2

3 g 2-Nitro-4-trifluormethyl- 4'-acetylamino-diphenylamin wurden mit 50 g konzentrierter Schwefelsäure 5 Stunden auf 140 °C erhitzt. Nach dem Abkühlen wurde das Reaktionsgemisch mit Wasser verdünnt und mit Natronlauge neutralisiert (bis pH = 7). Der gebildete Niederschlag wurde abfiltriert und im Vakuum bei 80 °C getrocknet. Es wurden 2,0 g 2-Nitro-4-trifluormethyl- 4'-aminodiphenylamin erhalten.

Stufe 3

3,6 g 2-Nitro-4-trifluormethyl- 4'-aminodiphenylamin wurde in 100 ml Ethanol in Gegenwart von 0,2 g Palladium (5%ig) auf Aktivkohle katalytisch hydriert. Nach beendeter Wasserstoff-Aufnahme wurde der Katalysator durch Filtration abgetrennt und das Filtrat nach Zusatz von 2 ml konz. HCl zur Trockene eingeengt.

Es wurden beige Kristalle mit einem Schmelzpunkt von 320 °C erhalten.

2,6-Diamino-4-trifluormethyl- 4'-aminodiphenylamin- trihydrochlorid (K2)

Stufe 1

Eine Mischung aus 12 g (0,04 Mol) 3,5-Dinitro-4-chlor-benzotrifluorid, 6,7 g (0,04 Mol) N-Acetyl-p-phenylendiamin, 4 g (0,05 Mol) Natriumhydrogencarbonat und 0,5 g Kupfer(I)-chlorid in 100 ml Nitrobenzol wurde 8 Stunden lang auf 170 °C erhitzt. Nach Aufarbeitung gemäss Stufe 1 von K1 wurden orange Kristalle von 2,6-Dinitro- 4-trifluormethyl- 4'-acetylamino- diphenylamin mit einem Schmelzpunkt von 255–257 °C erhalten.

Stufe 2

Das Produkt aus Stufe 1 wurde analog Stufe 2 von K1 mit konzentrierter Schwefelsäure behandelt und aufgearbeitet. Es wurden gelbe Kristalle von 2,6-Dinitro- 4-trifluormethyl- 4'-aminodiphenylamin mit einem Schmelzpunkt von 162–165 °C erhalten.

Stufe 3

Das Produkt aus Stufe 2 wurde analog Stufe 3 von K1 katalytisch hydriert und aufgearbeitet. Es wurden dunkelbraune Kristalle mit einem Schmelzpunkt von 180 °C (unter Zersetzung) erhalten.

2,4-Diamino- 6-trifluormethyl- 4'-aminodiphenylamin- trihydrochlorid (K3)

Stufe 1

12 g (0,04 Mol) 2-Chlor-3,5-dinitrobenzotrifluorid, 6,7 g (0,04 Mol) N'Acetyl-p-phenylendiamin, 4 g (0,05 Mol) Natriumhydrogencarbonat und 0,5 g Kupfer(I)-chlorid wurden in 100 ml Nitrobenzol 8 Stunden lang auf 170 °C erhitzt. Nach Aufarbeitung gemäss Stufe 1 von K1 wurden hellbraune Kristalle von 2,4-Dinitro- 6-trifluormethyl- 4'-acetylamino- diphenylamin mit einem Schmelzpunkt von 164–169 °C erhalten.

Stufe 2

Das Produkt aus Stufe 1 wurde analog Stufe 2 von K1 mit konzentrierter Schwefelsäure behandelt und aufgearbeitet. Es wurden gelbe Kristalle von 2,4-Dinitro- 6-trifluormethyl- 4'-aminodiphenylamin mit einem Schmelzpunkt von 224 °C (unter Zersetzung) erhalten.

Stufe 3

Das Produkt aus Stufe 2 wurde analog Stufe 3 von K1 katalytisch hydriert und aufgearbeitet. Es wurden violette Kristalle mit einem Schmelzpunkt von 205 °C (unter Zersetzung) erhalten.

Anwendungsbeispiele

Es wurden erfindungsgemässe Haarfärbemittel in Form einer Haarfärbe-Cremeemulsion der folgenden Zusammensetzung hergestellt:

| | |
|---|---|
| Fettalkohol $C_{12}$–$C_{18}$ | 10 |
| Fettalkohol $C_{12}$–$C_{14}$ + 2 EO-sulfat, Na-Salz, 28%ig | 25 |
| Wasser | 60 |
| 1. Oxidationsfarbstoffvorprodukt (Komponente E) | 7,5 mMol |
| Aminodiphenylamin (Komponente K) | 7,5 mMol |
| $Na_2SO_3$ (Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3%iger Wasserstoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90% ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschliessend getrocknet.

Als Aminodiphenylamin (Komponente K) wurden die Produkte K1, K2 und K3 (gemäss Herstellungsbeispielen) eingesetzt.

Als 1. Oxidationsfarbstoffvorprodukt (Komponente E) wurden die folgenden Verbindungen eingesetzt:

E 1: p-Phenylendiamin

E 2: N-Ethyl-N-(2-hydroxyethyl)-p-phenylendiamin

E 3: N.N-Bis-(2-hydroxyethyl)-p-phenylendiamin

E 4: 2-Chlor-p-phenylendiamin

E 5: p-Aminophenol

E 6: 2,5-Diaminoanisol

E 7: 2,4,5,6-Tetraaminopyrimidin

Die mit diesen Oxidationsfärbemittelvorprodukten erhaltenen Haaranfärbungen sind der folgenden Tabelle zu entnehmen:

Tabelle

| Anwendungs Beispiel | Komponente K | Komponente E | erhaltener Farbton |
|---|---|---|---|
| A 1 | K 1 | E 1 | dunkelrubin |
| A 2 | K 1 | E 3 | dunkelbraun |
| A 3 | K 1 | E 4 | rotbraun |
| A 4 | K 1 | E 5 | rehbraun |
| A 5 | K 1 | E 6 | rehbraun |
| A 6 | K 1 | E 7 | braun |
| A 7 | K 2 | E 1 | dunkelrubin |
| A 8 | K 2 | E 2 | dunkelbraun |
| A 9 | K 2 | E 5 | dunkelbraun |
| A 10 | K 3 | E 1 | dunkelrubin |
| A 11 | E 3 | E 2 | dunkelbraun |
| A 12 | K 3 | E 3 | dunkelbraun |
| A 13 | K 3 | E 4 | dunkelbraun |
| A 14 | K 3 | E 5 | rotbraun |
| A 15 | K 3 | E 6 | rotbraun |
| A 16 | K 3 | E 7 | rotbraun |

## Patentansprüche

1. Aminodiphenylamine der allgemeinen Formel I

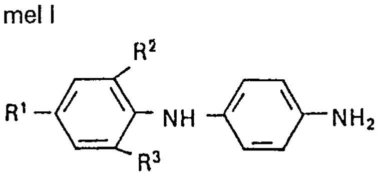 (I)

in der $R^1$, $R^2$ und $R^3$ Wasserstoff, eine Alkylgruppe mit 1–4 C-Atomen, eine $NH_2$-Gruppe oder eine $CF_3$-Gruppe sind, wobei eine dieser Gruppen die $CF_3$-Gruppe und wenigstens eine die $NH_2$-Gruppe darstellt.

2. 2,6-Diamino-4-trifluormethyl- 4'-aminodiphenylamin.

3. Verfahren zur Herstellung von Aminodiphenylaminen der allgemeinen Formel I nach Anspruch 1, dadurch gekennzeichnet, dass man eine Verbindung der allgemeinen Formel II

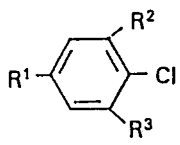 (II)

in welcher $R^1$, $R^2$ und $R^3$ Wasserstoff, eine Alkylgruppe mit 1–4 C-Atomen, eine $NO_2$-Gruppe oder eine $CF_3$-Gruppe sind, wobei eine dieser Gruppen die $CF_3$-Gruppe und wenigstens eine die $NO_2$-Gruppe darstellt, mit N-Acetyl-p-phenylendiamin in Gegenwart von Natriumhydrogencarbonat und Kupfer(I)-chlorid in einem polaren, inerten Lösungsmittel umsetzt, von dem entstehenden N-Acetyl-nitrodiphenylamin die Acetylgruppe abspaltet und das Nitrodiphenylamin katalytisch zum Aminodiphenylamin hydriert.

4. Verwendung der Aminodiphenylamine gemäss Anspruch 1 oder 2 oder deren Salze als Oxidationsfarbstoffvorprodukte in Haarfärbemitteln.

5. Haarfärbemittel, enthaltend Oxidationsfarbstoffvorprodukte in einem kosmetischen Träger, dadurch gekennzeichnet, dass als Oxidationsfarbstoffvorprodukte Aminodiphenylamine gemäss Anspruch 1 oder 2 oder deren Salze als Kupplersubstanzen und die in Oxidationshaarfärbemitteln üblichen Entwicklersubstanzen enthalten sind.

6. Haarfärbemittel nach Anspruch 5, dadurch gekennzeichnet, dass zusätzlich bekannte, weitere Kupplersubstanzen und direktziehende Haarfarbstoffe enthalten sind.

7. Haarfärbemittel nach Anspruch 5 oder 6, dadurch gekennzeichnet, dass die Aminodiphenylamine oder deren Salze in einer Menge von 0,05–10 Millimol pro 100 g enthalten sind.

## Revendications

1. Aminodiphénylamines de la formule générale I

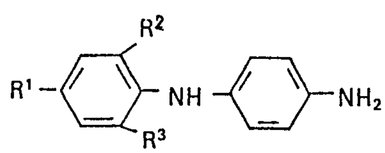 (I)

dans laquelle $R^1$, $R^2$ et $R^3$ représentent l'hydrogène, un groupe alkyle à 1 à 4 atomes de carbone, un groupe $NH_2$ ou un groupe $CF_3$ tandis que l'un de ces groupes représente le groupe $CF_3$ et au moins l'un d'eux le groupe $NH_2$.

2. 2,6-diamino-4-trifluorométhyl- 4'-aminodiphénylamine.

3. Procédé de production d'aminodiphénylamines de la formule générale I selon la revendication 1, caractérisé en ce que l'on fait réagir un composé de la formule générale II

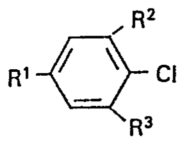

(II)

dans laquelle $R^1$, $R^2$ et $R^3$ représentent l'hydrogène, un groupe alkyle à 1 à 4 atomes de carbone, un groupe $NO_2$ ou un groupe $CF_3$ tandis que l'un de ces groupes représente le groupe $CF_3$ et au moins l'un d'entre eux le groupe $NO_2$, avec de la N-acétyl-p-phénylènediamine en présence de carbonate acide de sodium et de chlorure cuivreux dans un solvant polaire inerte, que l'on sépare de la N-acétyl-nitrodiphénylamine formée le groupe acétyle et que l'on hydrogène catalytiquement la nitrodiphénylamine en aminodiphénylamine.

4. Utilisation des aminodiphénylamines selon la revendication 1 ou 2 ou de leurs sels comme précurseurs de colorants d'oxydation dans des colorants pour cheveux.

5. Colorant pour cheveux contenant des précurseurs de colorants d'oxydation dans un support cosmétique, caractérisé en ce qu'il contient, comme précurseurs de colorants d'oxydation, des aminodiphénylamines selon la revendication 1 ou 2, ou leurs sels sous forme de substances de couplage et les substances de développement usuelles dans les colorants d'oxydation pour cheveux.

6. Colorant pour cheveux selon la revendication 5, caractérisé en ce que d'autres substances de couplage connues et des colorants pour cheveux s'appliquant directement sur la fibre sont également contenus.

7. Colorant pour cheveux selon la revendication 5 ou 6, caractérisé en ce que les aminodiphénylamines ou leurs sels sont présents en quantités de 0,05 à 10 mmoles par 100 g.

## Claims

1. Aminodiphenylamines corresponding to general formula I

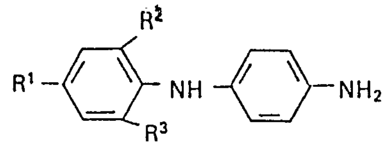

(I)

in which $R^1$, $R^2$ and $R^3$ represent hydrogen, a $C_1$–$C_4$ alkyl group, an $NH_2$ group or a $CF_3$ group, one of these groups being the $CF_3$ group and at least one the $NH_2$ group.

2. 2,6-Diamino-4-trifluormethyl- 4'-aminodiphenylamine.

3. A process for the preparation of the aminodiphenylamines of general formula I claimed in claim 1, characterized in that a compound corresponding to general formula II

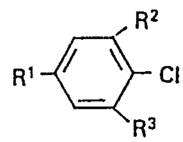

(II)

in which $R^1$, $R^2$ and $R^3$ represent hydrogen, a $C_1$–$C_4$ alkyl group, an $NO_2$ group or an $CF_3$ group, one of these groups being the $CF_3$ group and at least one being the $NO_2$ group, is reacted with N-acetyl-p-phenylendiamine in the presence of sodium hydrogen carbonate and copper(I) chloride in a polar inert solvent, the acetyl group is split off from the N-acetyl nitrodiphenylamine formed and the nitrodiphenylamine is catalytically hydrogenatted to the aminodiphenylamine.

4. The use of the aminodiphenylamines claimed in claim 1 or 2 or salts thereof as oxidation dye precursors in hair dyes.

5. Hair dyes containing oxidation dye precursors in a cosmetic carrier, characterized in that they contain as oxidation dye precursors the aminodiphenylamines claimed in claim 1 or 2 or salts thereof as coupler components and the developer components typical of oxidation hair dyes.

6. Hair dyes as claimed in claim 5, characterized in that known, other coupler components and substantive hair dyes are additionally present.

7. Hair dyes as claimed in claim 5 or 6, characterized in that the aminodiphenylamines or salts thereof are present in a quantity of from 0.05 to 10 millimol per 100 g.